# EUROPEAN PATENT APPLICATION

(11) **EP 2 503 340 A1**
(43) Date of publication of application: **26.09.2012**
(21) Application number: 11382076.5
(22) Date of filing: 22.03.2011
(51) Int. Cl.: G01N 33/68

(54) **Diagnostic marker for relapsing primary idiopathic focal segmental glomerulosclerosis**

(71) Applicant: Fundació Institut de Recerca Hospital Universitari Vall d'Hebron, Fundació Privada, 08035 Barcelona (ES)
(72) Inventor: López Hellín, Juan, 08036, BARCELONA (ES); Cantarell Aixendri, María del Carmen, 08036, BARCELONA (ES)
(74) Representative: ZBM Patents

(57) **Abstract**

The invention discloses an *in vitro* method for diagnosing relapsing idiopathic focal segmental glomerulosclerosis in a patient. The method is performed in urine and uses means to detect Apolipoprotein A-I.

## Description

The present invention provides methods and markers for the diagnosis and prognosis of primary focal segmental glomerulosclerosis (FSGS), namely of relapsing cases after kidney transplantation.

### BACKGROUND ART

Focal segmental glomerulosclerosis (FSGS) is a glomerular scarring disease characterized by an effacement of the podocyte foot when kidney biopsies are analyzed. Furthermore, when urine samples from patients suffering FSGS are analyzed, it is observed a massive urine protein lost, which progresses, at the end, to a renal failure.

Currently, there are two types of identified FSGS, primary and secondary FSGS, which depend on the cause resulting in the disease. In order to determine whether the subject is suffering from the primary or secondary FSGS, the nephrologyst evaluates the patient clinical history. Secondary FSGS appears as a natural consequence of other diseases (such as hypertension), and the nephrologists detects the onset when the nephrotic syndrome is established and the biopsy shows the FSGS pattern. On the contrary, primary FSGS appears without any associated cause and, in general during the childhood.

Regarding the primary FSGS, there is strong evidence supporting two different aetiologies: a form caused by genetic mutations which is associated to primary alterations of podocyte proteins, and an idiopathic form, of unknown origin, related to unknown plasma factors, probably proteins, that alter the glomerular permeability.

Secondary FSGS includes virus-associated FSGS, unilateral renal agenesis, and FSGS associated with renal dysplasia, surgical renal ablation, hypertension, and obesity. It develops as a maladaptive response to glomerular "overwork".

Idiopathic primary FSGS (by now on also termed idiopathic FSGS or simply

FSGS) has particularly the worst prognostic, even after kidney transplantation, in such a way that about 30-40 % of the patients newly develop the nephrotic syndrome. The relapsing appears from few hours to months or years post-transplantation. Therefore, it is of great importance to the nephrologists the identification of people suffering from said idiopathic FSGS.

It is known in the state of the art, that primary idiopathic FSGS differs from the secondary FSGS mainly in the following aspects: (a) the extent of the foot process effacement when the biopsy is analyzed; and (b) the clinical presentation. Regarding aspect (a), there is observed extensive foot process effacement when the subject is suffering from the idiopathic FSGS, whereas said effacement is in a less extent when the subject is suffering from secondary FSGS. Regarding aspect (b), patients suffering from idiopathic FSGS show a clinical presentation consistent with full-blown nephrotic syndrome, whereas in patients suffering from secondary FSGS it is observed a clinical presentation consistent with proteinuria without hypoalbuminemia.

The differences pointed out above between both types of FSGS, however, are under the subjective criteria of the physician (i.e., aspect (a) above) or the markers which are measured for determining the clinical presentation are not specific enough of the type of the disease (aspect (b)). Therefore, even existing differences between both types, the current criteria make difficult a correct diagnostic, and even a correct definition of primary idiopathic FSGS.

People suffering from FSGS are treated with steroids. However, current steroid-based treatments are not efficient and often they can give rise to the development of the steroid-resistant nephrotic syndrome (SRNS). An alternative treatment is based on the administration of the immunosuppressive cyclosporine A, which induces a remodelling effect on the glomerular proteins. However, about 60% of the patients do not respond to the treatment and they finally require dialysis and kidney transplantation due to renal failure. Therefore, there are not specific or efficient treatments for FSGS.

A severe problem related to this disease is the frequent relapse after kidney transplantation, sometimes few hours after the organ replacement. The relapse seems to be related to the idiopathic rather than to the genetic and secondary forms of FSGS, although a high recurrence has been associated to patients with one heterozygote *NPHS2* gene (podocin encoding gene) mutations or polymorphisms. The relationship between these mutations and the permeability factor has also been suggested.

As it has been stated above, primary FSGS is usually diagnosed by the histological findings in a biopsy from a subject suspected of suffering the disease. Biopsies, besides being an invasive procedure, can fail in detecting the scarring because the focal nature of the disease (i.e. by biopsing a nonaffected region of the kidney), and additionally, can not differentiate among secondary and primary FSGS, as well as between the idiopathic and genetic forms.

Another therapeutic approach for the idiopathic primary FSGS patients is by means of plasmapheresis, which is the removal and substitution of plasma proteins. Plasmapheresis treatment is based on the hypothesis that idiopathic FSGS is probably caused by a circulating plasma factor, which is deleted by means of this method of filtration.

In addition, all the therapeutic approaches based on the administration of immunosuppressant and plasmapheresis are directed to obtain transitory proteinuria improvements. They are not directed to the lesions occasioned in FSGS. The disease evolves by damaging kidney, and when the damage is so that kidney is not functional anymore, the subject (patient) is remitted to dialysis while waiting for a new kidney.

Nowadays, the relapse of FSGS after transplantation is suspected when the urine protein levels, which are determined regularly, increase over a certain value (e.g. 1 g/day). At this point, the facultative requires a kidney biopsy to diagnose the disease, with the above mentioned drawbacks

In view of the above, therefore, nephrologists do not dispose of markers allowing the diagnosis or prognosis of the relapsing of idiopathic primary FSGS in a simplified way. Additionally, due to the unknown aetiology of the pathology, no specific treatment for the same is available today.

In one attempt of providing a suitable marker, the Th2 soluble marker protein (ST2) has been proposed as marker for the diagnosis of relapsing (or recurrence) idiopathic nephrotic syndrome. Thus, as explained in Bruneau et al., "High levels of soluble ST2 protein in recurrence idiopathic nephrotic syndrome after kidney trasplantation", American Journal os Kidney Diseases - 2009, Vol. 54(3), pp. 522-32, the detection of ST2 in the serum of kidney transplanted patients is indicative of the relapsing of the corticoid-resistant FSGS (which is the same as idiopathic FSGS). The early detection of ST2 avoids biopsies and a fast therapeutic schedule may be determined, namely the performing of plasmapheresis. However, as stated by Bruneau et al., ST2 protein is a marker of idiopathic nephrotic syndrome, but it does not seem to be involved in the development of the disease.

Nonetheless, the marker proposed by Bruneau et al. *(supra)* is detected in the serum of the patients, with the drawbacks accompanying the extraction of blood, always aggressive for some patients, in special for children. Moreover, blood is known as the most complex fluid in mammals, thus being sometimes difficult to detect the presence of minor traces of a component, due to the presence of blood proteins, which can interfere in said detection.

Some urine markers for glomerular diseases have been previously disclosed. Examples of these are those disclosed by Shui et al., "Urinary proteome and potential biomarkers associated with serial pathogenesis steps of focal segmental glomerulosclerosis", Nephrol Dial. Transplant 2008, Vol. 23, pp.176-185; and Thongboonkerd et al., "Biomarker discoveries in glomerular diseases using urinary proteins", Proteomics Clin. Appl. 2008, Vol. 2, pp. 1413-1421.

Both documents show lists of putative urine biomarkers related with glomerular diseases such as FSGS, or glomerulonephritis. Some of the markers proposed are extracellular matrix protein 1, serotransferrin, haptoglobine, zinc-α2-glycoprotein, and glutathione -S-transferrase. None of the documents shows a marker suitable to early diagnose in urine, of recurrence of idiopathic FSGS.

Other document showing the urine protein composition of patients with nephrotic syndrome of different aetiologies is Candiano et al., "Proteins and protein fragments in nephrotic syndrome: Clusters, specificity and mechanisms", Proteomics Clin. Appl. 2008, Vol. 2, pp. 956-963. Candiano et al. analysed by 2D-electrophoresis the urine of several patients suffering from FSGS, membranous nephropathy (MN) and mTOR-induced proteinuria. They propose the detection of specific 2D- electrophoresis patterns in order to discriminate between tubular diseases (such as MN) and glomerular diseases (such as FSGS). In the same way as Shui et al. *(supra)* this document is silent about relapsing idiopathic FSGS.

In spite of the efforts made, there is the need in the state of the art of further markers for detecting the relapse or recurrence of idiopathic FSGS.

### SUMMARY OF THE INVENTION

With the aim of solving the problem mentioned before, the inventors performed a deep study of the body fluid proteins detectable in patients with nephrotic syndrome of different aetiologies. Surprisingly, they found that apolipoprotein A-I could be differentially detected in cases of relapsing idiopathic FSGS.

Thus in a first aspect the invention relates to an *in vitro* method for diagnosing relapsing idiopathic focal segmental glomerulosclerosis (FSGS) in a subject suspected of suffering it, comprising the step of detecting the presence of apolipoprotein A-I in a test sample of said subject, wherein the detection of the protein is indicative of relapsing idiopathic focal segmental glomerulosclerosis.

Apolipoprotein A-I (hereinafter also referred as "ApoA-I") is the major protein component of high density lipoprotein (HDL) in plasma. Human ApoA-I is identified in the UniProt database with the number P02647 of 21-July-1986 (Version 1). Chylomicrons secreted from the intestinal enterocyte also contain ApoA-I but it is quickly transferred to HDL in the bloodstream. The protein promotes cholesterol efflux from tissues to the liver for excretion. It is a cofactor for lecithin cholesterolacyltransferase (LCAT) which is responsible for the formation of most plasma cholesteryl esters. It has an approximate molecular weight of 28 KDa.

By means of the *in vitro* method according to the invention, the detection is made in test samples being bodily fluid samples, thus avoiding unnecessary kidney biopsies. This is of special relevance in those patients which have already been submitted to a prior biopsy on one hand, because a second and subsequent surgical intervention implies a risk in itself and, on the other hand, because it has been observed that second biopsies in kidney lead in a great percentage to kidney complications.

Another advantage of the *in vitro* method according to the invention is the reduction of false negative determinations when biopsy samples are analyzed. These false negative are subjects with FSGS not detectable due to the focal nature of the disease. In other words, they are not detected as such if the retrieved sample does not contain the morphology of a FSGS kidney.

In addition to the above, the early detection of a putative relapse allows the facultative to establish quickly a therapeutic approach in order to avoid a further renal failure requiring a subsequent transplantation. The *in vitro* method according to the invention is a fast method if compared also with those methods based on obtaining biopsies, these latter being time consuming due to the tissue manipulation which, in turn, is always a complex task.

A second aspect of the invention is a method of deciding or recommending whether to iniciate pharmacological treatment of a subject suspected of suffering a relapsing of idiopathic focal segmental glomerulosclerosis, which method comprises the steps of:
a) detecting, in vitro, the presence of ApoA-I in a test sample of the subject; and
b) diagnosing the relapsing of idiopathic focal segmental glomerulosclerosis if ApoA-I is detected;
wherein:
ca) if the subject is diagnosed of suffering a relapsing of idiopathic focal segmental glomerulosclerosis, then the initiation of apheresis is recommended, and
cb) if the patient is diagnosed of not suffering a relapsing of idiopathic focal segmental glomerulosclerosis, the follow-up of renal function and proteinuria is performed,
   optionally in consideration of the result of an examination of the patient by a nephrologist.

A third aspect of the invention is the use of means for detecting the presence of apolipoprotein A-I in a body fluid sample for the diagnosis of relapsing idiopathic focal segmental glomerulosclerosis in the methods of the first aspect of the invention.

A fourth aspect of the invention relates to the use of ApoA-I as clinical diagnostic marker of relapsing idiopathic FSGS.

Another aspect of the invention is a means for extracting ApoA-I from a blood sample, including serum and plasma, for use in the treatment of idiopathic focal segmental glomerulosclerosis.

This aspect could be also formulated; as the use of means for extracting ApoA-I from a blood sample for the preparation of a medicament for the prophylaxis and/or the treatment of idiopathic focal segmental glomerulosclerosis. In other words, the invention elicits a method of treatment of FSGS in which the blood of a patient is extracted with means able to extract ApoA-I from the blood sample (serum, plasma, entire blood, etc.).

### BRIEF DESCRIPTION OF THE DRAWINGS

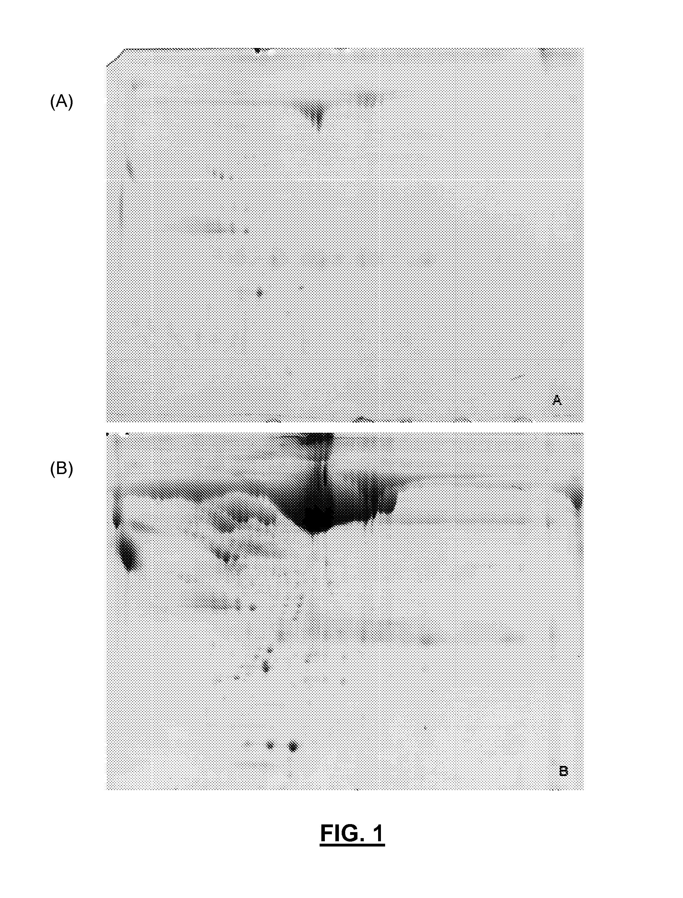

FIG. 1, related to Example 1, is an image of a two dimensional electrophoresis of pooled urine samples with standard tris-glycine buffer. (A): group NR (non relapsing FSGS); (B): group R (relapsing FSGS). Gels were stained with colloidal coomassie, digitized with a calibrated densitometer (GS800 Bio-Rad, Hercules, Ca, USA) and the images analyzed by PDQuest® 6.2 software (Bio-Rad Laboratories, Hercules, CA, USA).

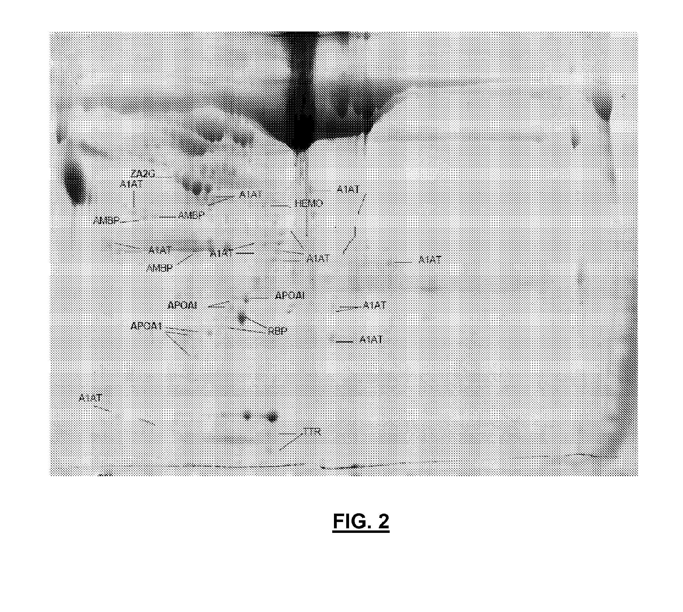

FIG. 2, related to Example 1, is a two dimensional electrophoresis image wherein the identified differential spots in relapsing patients vs. non relapsing patients are showed: 18 spots correspond to α-1 antitrypsin (A1AT), 3 spots are protein AMBP (uniprot code P02760), 1 spot is hemopexin (HEMO), 2 spots are transthyretin (TTR), 2 spots correspond to retinol binding protein (RBP), 1 spot is zinc α-2-glicoprotein (ZAG), and 6 spots are apolipoprotein A-I (apoA I). The gel was stained and digitized as in FIG. 1.

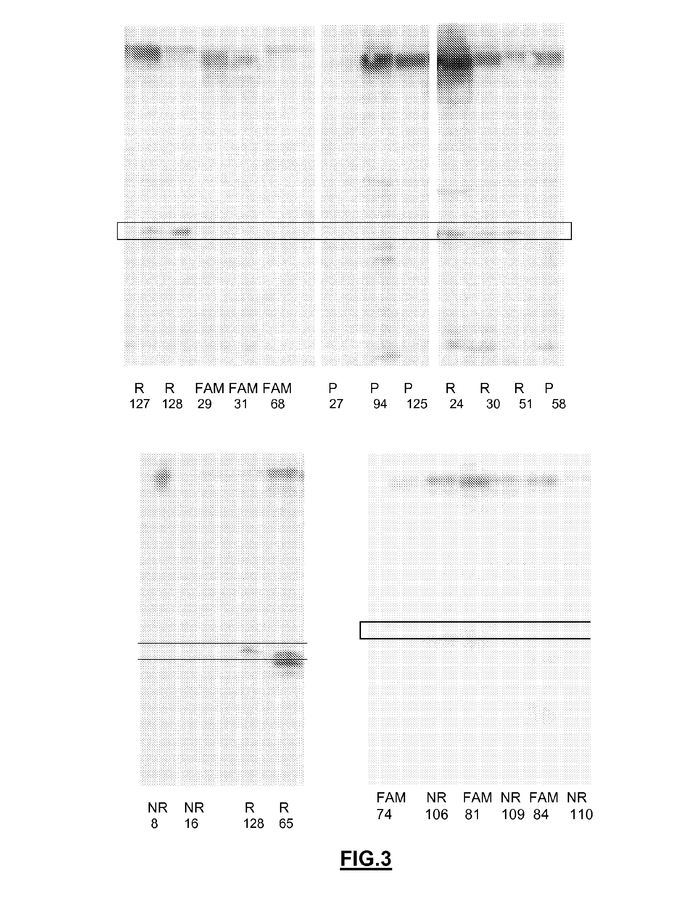

FIG. 3, relating to Example 2, is an image of a Western blot immunoassay of ApoA-I (visualized into the square) in concentrated urine from different patients: R relapsing FSGS, NR non relapsing FSGS, FAM familiar FSGS, P FSGS-unrelated proteinuria. Sixty µg of protein were applied to each well. The boxes show the zone where ApoA-I should appear. Detection was performed and probed with anti ApoA-I (goat polyclonal PAB9966 Abnova, Jhongli, Taiwan) and revealed with chemiluminescence Kit (ECL plus or ECL, Amersham Pharmacia Biotech). Visualization was done using a Fujifilm LAS3000 chemiluminescence detector. Numbers under each lane indicates the patient reference.

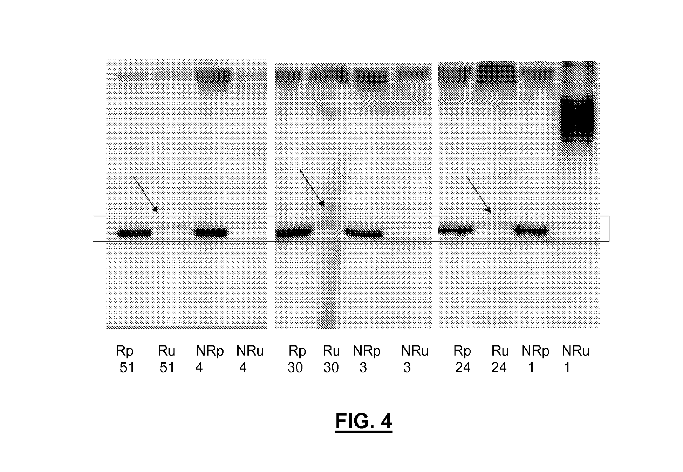

FIG. 4, related to Example 2, shows the electrophoretic bands corresponding to Apo A-I (arrow and square) in urine from 3 different relapsing patients (Ru) in comparison with the ApoA-I from their paired plasma samples (Rp), and with Apo A-I from other plasma and urine samples of non-relapsing patients (NRp, NRu). Numbers under each lane indicates the patient reference.

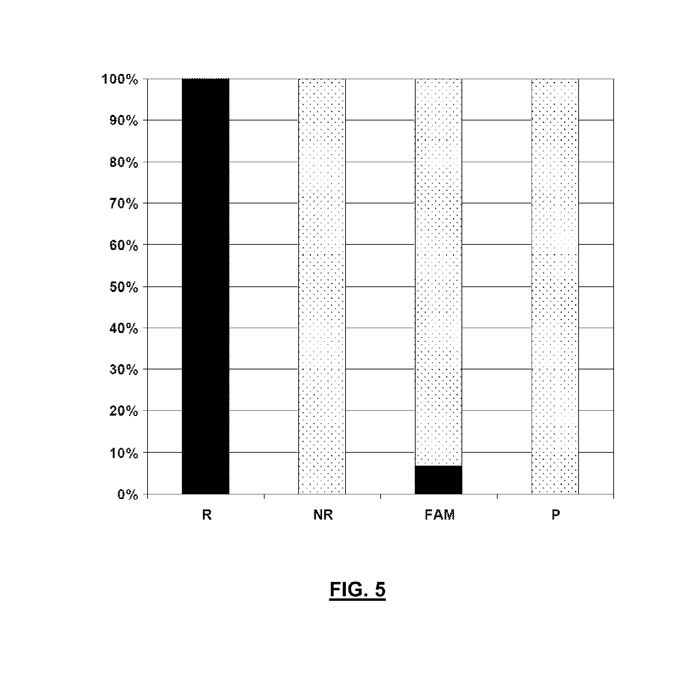

FIG. 5, related to Example 2, is a bar diagram showing the distribution of apolipoprotein A-I results (in %) for every studied group. Black: positive; dotted: negative. R, relapse; NR, no relapse; P, non-related FSGS proteinuria.

### DETAILED DESCRIPTION OF THE INVENTION

The *in vitro* method according to the invention comprises the step of detecting the presence of ApoA-I in a test sample of the subject suspected of suffering FSGS. As will be illustrated below by means of examples, the mere presence of the protein in the sample is indicative of relapsing idiopathic FSGS, especially when the body fluid is urine. This is so, since the patients with other glomerular diseases do not have this protein in the urine.

In a preferred embodiment of the first aspect of the invention, the method further comprises, once confirmed the presence of ApoA-I in the sample, the steps in the specific order of measuring the amount of ApoA-I in the test sample, and then comparing the amount of ApoA-1 with a reference value from a sample, of a subject previously diagnosed of relapsing FSGS.

In other words, the amount of ApoA-I is compared with a reference pattern, previously established from data of subjects diagnosed of relapsing FSGS. The pattern may include several ranges of concentrations, being each range associated, for example to a determined manifestation or severity of the disease. This measure allows determining the relapsing stage or gravity.

Illustrative non-limitative examples of test samples include urine, blood, serum, plasma. In a preferred embodiment of the first and second aspects of the invention, the test sample is urine.

In one embodiment of the first aspect of the invention, the ApoA-I protein determination may be preferably performed in urine as test sample. This aspect implies the additional advantage of avoiding the blood extraction. Blood extraction is always aggressive, especially for children, who unfortunately represent a high percentage of the FSGS patients. Additionally, the detection in urine, which is a simpler fluid than blood, represents a more feasible intervention over the patient. Urine samples may be collected every day after kidney transplantation and therefore, a fine progression of the postoperative may be reported.

In another preferred embodiment of the first aspect of the invention, the diagnosis of relapsing idiopathic focal segmental glomerulosclerosis is made in the test sample of a subject which has been kidney transplanted.

It is to be noted that idiopathic FSGS is treated (although not specifically) by means of cyclosporine A or plasmapheresis, and it is tried to avoid kidney transplantation by preserving the health of the kidney. Nonetheless if, further to the treatment, ApoA-I is detected in urine, this means that the relapse is starting or prosecuting. Therefore, the presence and the specific levels of

ApoA-I allow the control of the progression of the disease.

Another advantage of the diagnostic method according to the invention is that it not only provides a fast diagnosis of the recurrence of idiopathic FSGS in cases were kidney transplantation has already been carried out. Indeed, it also represents the confirmation that a particular patient was really suffering from idiopathic FSGS and no other nephrotic syndrome with proteinuria. This fact is of special relevance, since it allows the facultative a proper therapeutic approach, avoiding those that are not effective for FSGS (corticoid treatment), as well as the secondary adverse effects thereof.

Preferred means used for detecting the presence of apolipoprotein A-I in a test sample for the diagnosis of relapsing idiopathic focal segmental include, without limitation, antibodies or fragments thereof, which specifically bind to Apo A-I.

For the purposive of understanding, the following definitions are included.

The "relapsing or recurrence" of a disease, in this case of the idiopathic focal segmental glomerulosclerosis, relates to the new manifestation of a previously diagnosed disease after a periof wherein no symptoms were detected. Etymologically, it means "who falls again", and occurs when a person is affected again by a condition that affected him in the past.

The "test sample" refers to a sample of bodily fluid obtained for the purpose of diagnosis, prognosis, or evaluation of a subject of interest, such as a patient. In certain embodiments, such a sample may be obtained for the purpose of determining the outcome of an ongoing condition or to determine whether a pharmacological treatment should be applied to a subject.

The term "diagnosis" is known to the person skilled in the art. Diagnosing is understood as becoming aware of a particular medical condition, disease, complication or risk.

The term "subject" used herein and according to the present invention relates to a healthy individual, an apparently healthy individual or particularly an individual suffering from a disease (patient). The patient can be both a male and a female individual; particularly the patient is suffering from FSGS.

By "renal function" is to be understood in nephrology, the indication of the state of the kidney and its role in renal physiology. Glomerular filtration rate (GFR) describes the flow rate of filtered fluid through the kidney. Creatinine clearance rate (CCr or CrCl) is the volume of blood plasma that is cleared of creatinine per unit time and is a useful measure for approximating the GFR. Both GFR and CCr may be accurately calculated by comparative measurements of substances in the blood and urine, or estimated by formulas using just a blood test result (eGFR and eCCr). The results of these tests are important in assessing the excretory function of the kidneys.

"Proteinuria" is the presence of an excess of serum proteins in the urine. Proteinuria may be due to disease in glomerulus, increased quantity of proteins in serum (overflow proteinuria), or due to low reabsorbtion at proximal tubule (fanconi).

The term "apheresis" encompasses a medical technology in which the blood of a donor or patient is passed through an apparatus that separates out one particular constituent and returns the remainder to the circulation. It is thus an extracorporeal therapy. If plasma is separated, then the apheresis is called plasmapheresis. If lipids are separated, then the apheresis is called lipid apheresis, for example in LDL apheresis the low-density lipoproteins are separated from the blood. If platelets are removed, then apheresis is called platelepheresis. Other types of apheresis are leukapheresis (leukocytes are extracted) or erythrocytapheresis (erythrocytes are separated).

As "antibody or fragment thereof specifically binding ApoA-I" is to be undestood any immunoglobulin or fragment of the same able to bind the antigen defined by the apolipoprotein A-I. It includes monoclonal and policlonal antibodies. The term "fragment of an antibody" encompasses any part of an antibody having the size and conformation suitable to bind an epitope of ApoA-I. Suitable fragments include F(ab), F(ab') and Fv. An "epitope" is the part of the antigen being recognized by the immune system (B-cells, T-cells or antibodies). Suitable antibodies may be those mentioned in the examples, such as the goat polyclonal PAB9966 Abnova, Jhongli, Taiwan. Other usable antibodies include Chemicon-Millipore AB740 polyclonal, Chemicon-Millipore MAB010/A11 monoclonal, Raybiotech DS-MB-00089, SantaCruz sc-69755 monoclonal, Thermo-Pierce PA5-14310 polyclonal, Genway 18-464-436030 monoclonal.

In a preferred embodiment of the third aspect of the invention the antibody or fragment thereof for detecting ApoA-I is included in a kit. The kit may additionally comprise the means (additives, solvents) to visualize the antigen-antibody interactions (dipsticks, chemiluminescent reagents, turbidimetric reagents, etc.). Suitable additives, solvents and reagents to visualize the antigen-antibody interaction are disclosed in the examples.

The means used for detecting the presence of apolipoprotein A-I for the purpose of the invention may also be integrants or reagents of different commercial kits, or the combination of an antibody or of antibodies with reagents of other kits, for example to visualize the antigen-antibody interaction. Commercial kits can be, for example the ELISA Abnova kit (Ref. KA), a sandwich enzyme for quantitative determination of human ApoA-I. Other usable ELISA kits include Elab&USCNLIFE (Ref. E0604h), GenWay Biotech (Ref. 40-288-20069-ELISA_Kits), Mabtech AB, (Ref 3710-1A-20).

ApoA-I detection may also be performed with standard clinical analyzers, such as the Siemens (Dade-Behring) BN ProSpec®.

In a preferred embodiment of the invention ApoA-I is used as urine clinical diagnostic marker of relapsing idiopathic FSGS.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### Example 1. Two-dimensional proteomic analyses for plasma and urine.

### Patients:

Blood and urine samples were obtained from 55 patients after kidney transplantation. The cause of transplantation was in 51 cases primary FSGS, and in 4 cases other kidney diseases unrelated to FSGS. According with the diagnosis and the clinical evolution after the transplantation, the patients were divided into 5 groups: Group R (9 patients) relapsing (or recurrence) of idiopathic FSGS during the first year after transplantation, with proteinuria higher than 3g/day, and FSGS positive biopsy. Group NR (27 patients), nonrecurring idiopathic FSGS, with normal proteinuria for more than one year after transplantation. Group FAM (15 patients), familiar (genetic) FSGS with normal proteinuria after transplantation. Group P (4 patients), with proteinuria unrelated to FSGS was designed to test the specificity of the findings (Control group type 2). The protocol for sample collection was approved by the hospital ethics committee, and informed consent was obtained from the patients. The study was conducted in accordance with the principles of the Declaration of Helsinki.

### Samples were processed as follows:

Blood samples were collected in EDTA and centrifuged 10 minutes at 1200xg. Urine samples maintained in ice were centrifuged 10 minutes at 1200xg at 4°C. Blood plasma and urine supernatants were stored frozen in the participating hospitals at -20°C during less than 1 month. Samples were shipped in dry ice from the hospitals to Vall d'Hebron Institute of Research, and stored at -80°C until processing. Urine was thawed and centrifuged a second time, discarding the precipitated salts. Fifteen milliliters of urine were concentrated by ultrafiltration (Amicon Ultra 3K, cut-off of 3kDa) to a volume of 500 µl, thus concentrating 30 fold the proteins over 3k, with no salt concentration. Pools of plasma and urine were prepared for each study group defined for the proteomic analyses.

For determining the protein concentration in urine samples the bicinconinic acid method was used (Pierce BCA, thermo-Fischer Scientific, Rockford, IL, USA).

### Two dimensional electrophoresis:

Two-dimensional electrophoresis of pooled urine was run as: 10 µL of concentrated urine in 450 µL of rehydration solution (8M urea; 2% CHAPS; 1.2% hydroxiethyl disulfide; 0.5% IPG ampholine buffer pH3-10 and 0.002% bromophenol blue) were spread on a 24-cm ceramic holder (Amersham Biosciences Europe, Freiburg, Germany) with a 24-cm strip of polyacrylamide gel with immobilized pH gradient (Immobiline DryStrip 3-10L, Amersham Biosciences Europe, Freiburg, Germany. The isoelectric focusing was run in an IPGphor™ system (Amersham Biosciences Europe, Freiburg, Germany). Focused gel strips were equilibrated for 15 minutes with 10 ml of reducing SDS buffer (50mM Tris, 6M urea, 30% glycerol, 2% SDS, 1% DTT, 0.002% bromophenol blue) and 15 minutes more with 10 ml of alkylating SDS buffer (same without DTT and with 4% iodoacetamide). The equilibrated strips were placed over a 1 mm, 16cm x 24cm SDS-PAGE gel (12.5%T, 2.6%C) and were run in a Ettan Dalt 6 system (Amersham Biosciences Europe, Freiburg, Germany), using either conventional tris-glycine-SDS buffer or tris-tricine-SDS buffer, for 4.5 hours at 100 Watts. Gels were stained with colloidal coomassie, digitized with a calibrated densitometer (GS800 Bio-Rad, Hercules, Ca, USA) and the images analyzed by PDQuest® 6.2 software (Bio-Rad Laboratories, Hercules, CA, USA).

FIG. 1 shows the two dimensional electrophoresis of pooled urine samples of non relapsing FSGS (NR, panel A) in comparison with relapsing FSGS (R3, panel B). To detect kidney specific proteins, or kidney modified proteins, the spots in R urine also present in R plasma (previously detected in a two dimensional electrophoresis of plasma samples run in the same conditions) were subtracted. With such a practice, those proteins that leaked directly from blood trough the damaged glomerular barrier were eliminated from the differential protein set. The differential analysis detected (after discarding the spots matched to plasma spots) 41 differential spots, and the mass spectrometric analysis identified 6 different proteins in 33 of these spots. In FIG. 2, which is the image of the two dimensional electrophoresis of R, the differential proteins are depicted and duly identified. The differential proteins detected were α-1 antitrypsin (A1AT), Alpha-1-microglobulin/bikunin precursor (AMBP), hemopexin (HEMO), transthyretin (TTR), retinol binding protein (RBP), zinc α-2-glicoprotein (ZAG), and apolipoprotein A-I (ApoA-I).

From these proteins, and after deep analysis of its identity, the inventors considered zinc α-2-glicoprotein (ZAG) and apolipoprotein A-I (ApoA-I) as being of special interest. The other differential proteins found (alpha-1-antitrypsin, protein AMBP, retinol binding protein, transthyretrin and hemopexin) have been previously described in urine and are rather unspecific.

It is interesting to note that, while differential ZAG appears only in one spot, ApoA-I appears in six different forms: 1 of the expected molecular weight, 3 under (probably fragments) and 2 over the theoretical molecular weight. One of the latter is also the most intense ApoA-I spot.

As illustrated in next Example 2, the inventors surprisingly found that ApoA-I was differentially detectable in the urine samples of relapsing idiopathic FSGS, and was apparently not present in the other groups.

In relation to ZAG in urine, it was not detected in non-concentrated urine. However, 1 µL of urine concentrated as previously described was enough to allow the detection of ZAG in all the patients studied, regardless of the group to which were ascribed (data not shown).

### Example 2 Individual determination of apolipoprotein A-I in urine

Detection of ApoA-I in urine was achieved applying an amount of concentrated urine containing 60 µg of protein. The results of the western blot performed are depicted in FIG. 3. In these conditions, a band of variable intensity was observed in all the relapsing patients (R). So then, ApoA-I could be detected.

However, the most interesting point derivable from FIG. 3 is the no presence of ApoA-I band neither in the no-relapsing patients (NR) studied nor the familiar FSGS (FAM).

For the performance of the western blot of FIG. 3, analysis was done in 5x7 cm SDS-PAGE gels with standard tris-glycine-SDS buffer. Proteins in gels were transferred to PVDF membranes, blocked with 5% skimmed milk in TBST (Tris-HCl 10 mM, NaCl 100 mM, Tween 20 0,1%, pH 7,5) and probed with either anti ApoA-I (goat polyclonal PAB9966 Abnova, Jhongli, Taiwan) 1:5000 in TBST. After washing the membrane with TBST, they were incubated 1 h at room temperature with appropriate horseradish peroxidase-conjugated secondary antibody: rabbit anti goat 1:2.000 for ApoA-I (P0449 Dako, Glostrup, Denmark), washed again with TBST and developed using an enhanced chemiluminescence Kit (ECL plus or ECL, Amersham Pharmacia Biotech), and visualized using a Fujifilm LAS3000 chemiluminescence detector. Best results were obtained with ECL, using 5 minutes for ApoA-I. Bands were quantified using the QuantityOne software (Bio-rad, Hercules, CA, USA) and statistical comparisons were done with the StatGraphics statistical package (Manugistics Inc, Herndon, VA, USA).

To check if this finding was specific for relapsing idiopathic FSGS, the inventors studied 4 patients with proteinuria not related to FSGS (group P), and with a wide range of proteinuria, from 0.7 to 9.4 g/day. Using the same procedure than for the other patients (60 µg of concentrated urine) a western blot was realized after electrophoresis. FIG. 4 shows the results of the analysis. There are illustrated the bands appearing in plasma (Rp) and urine (Ru) of 3 patients with relapsing FSGS, in comparison with plasma and urine samples of non-relapsing FSGS (NRp and NRu). In none of these patients (NRu) appeared the ApoA-I band in these conditions.

An intriguing characteristic derivable from FIG. 4 is that the ApoA-I band detected in the urine of the relapsing idiopathic FSGS patients is that it seems to have an apparent molecular weight slightly higher that the regular ApoA-I found in plasma, and higher than the weak bands that appear in some FSGS-unrelated proteinuria when overexposed. This observation fully agrees with the more abundant differential isoform found in the proteomic analyses disclosed before, which has an apparent molecular weight somewhat higher than the main ApoA-I form found in plasma. All these observations reinforce the differential character of the apolipoprotein A-I as diagnostic marker and allow the formulation of the hypothesis that this isoform could be a specific isoform of the idiopathic FSGS, and probably involved in the etiology of the disease.

Therefore, those means, such as specific antibodies or fragments raised against ApoA-I, that can serve to extract this protein from the blood of a patient may be used for treating the disease. These antibodies are usually immobilized in a support (such as beads) and absorb the specific ApoA-I protein (antigen) to which they are rose.

Finally, the data from all the studied patients are summarized in FIG. 5. This bar diagram shows the positive (black) and negative (dotted) percentages of patients containing ApoA-I in the urine. As can be seen, all the patients with relapsing idiopathic FSGS were positive for ApoA-I, meanwhile the non-relapsing patients previously transplanted for suffering idiopathic FSGS were negative regarding the presence of ApoA-I in urine.

All these data taken together demonstrate that the determination of ApoA-I in urine is a specific biomarker or diagnostic marker of idiopathic FSGS, which is recurring after transplantation. The early detection of the relapse may be of great help in order to attend the patient in the most suitable therapeutic pattern, avoiding biopsies in a first instance and minimizing or prolonging the need of subsequent kidney transplantation.

### REFERENCES CITED IN THE APPLICATION

- Bruneau et al., "High levels of soluble ST2 protein in recurrence idiopathic nephrotic syndrome after kidney trasplantation", American Journal os Kidney Diseases - 2009, Vol. 54(3), pp. 522-32.
- Shui et al., "Urinary proteome and potential biomarkers associated with serial pathogenesis steps of focal segmental glomerulosclerosis", Nephrol Dial. Transplant - 2008, Vol. 23, pp.176-185.
- Thongboonkerd et al., "Biomarker discoveries in glomerular diseases using urinary proteins", Proteomics Clin. Appl. - 2008, Vol. 2, pp. 1413-1421.
- Candiano et al., "Proteins and protein fragments in nephrotic syndrome: Clusters, specificity and mechanisms", Proteomics Clin. Appl. - 2008, Vol. 2, pp. 956-963.

## Claims

1. An *in vitro* method for diagnosing relapsing idiopathic focal segmental glomerulosclerosis (FSGS) in a subject suspected of suffering it, comprising the step of detecting the presence of apolipoprotein A-I in an test sample of said subject, wherein the detection of the protein is indicative of relapsing idiopathic focal segmental glomerulosclerosis.

2. The *in vitro* method of claim 1, which further comprises, once confirmed the presence of ApoA-I in the sample, the following steps in the specific order:
- measuring the amount of ApoA-I in the test sample, and then
- comparing the amount of ApoA-I with a reference value from a sample, of a subject previously diagnosed of relapsing FSGS.

3. The *in vitro* method according to any of claims 1-2, wherein the test sample is urine.

4. The *in vitro* method according to any of claims 1-3, wherein the sample is from a patient already submitted to a prior kidney transplantation.

5. A method of deciding or recommending whether to iniciate pharmacological treatment of a subject suspected of suffering a relapsing of idiopathic focal segmental glomerulosclerosis, which method comprises the steps of:
a) detecting, in vitro, the presence of ApoA-I in a test sample of the subject; and
b) diagnosing the relapsing of idiopathic focal segmental glomerulosclerosis if ApoA-I is detected;
wherein:
ca) if the subject is diagnosed of suffering a relapsing of idiopathic focal segmental glomerulosclerosis, then the initiation of apheresis is recommended, and
cb) if the patient is diagnosed of not suffering a relapsing of idiopathic focal segmental glomerulosclerosis, the follow-up of renal function and proteinuria is performed,
optionally in consideration of the result of an examination of the patient by a nephrologist.

6. The method of claim 5, wherein the test sample is urine.

7. Use of means for detecting the presence of apolipoprotein A-I in a test sample for the diagnosis of relapsing idiopathic focal segmental glomerulosclerosis in any of the methods of claims 1 to 6.

8. The use of claim 7, wherein the diagnostic means is an antibody or a fragment thereof which specifically binds to Apo A-I.

9. The use of claim 8, wherein the antibody or fragment thereof, forms part of a kit.

10. Use of Apo A-I as clinical diagnostic marker of relapsing idiopathic FSGS.

11. The use of claim 8, as clinical diagnostic marker in a urine sample.
